(19) **Europäisches Patentamt**

**European Patent Office**

**Office européen des brevets**

(11) Veröffentlichungsnummer: **0 515 939 B1**

(12) **EUROPÄISCHE PATENTSCHRIFT**

(45) Veröffentlichungstag der Patentschrift: **06.12.95**

(51) Int. Cl.6: **D01G 9/08**, D01G 11/00, D01G 21/00, D01G 23/02, D01G 23/08, D01G 37/00

(21) Anmeldenummer: **92108319.2**

(22) Anmeldetag: **16.05.92**

(54) **Vorrichtung zum Auflösen von Flockenverklumpungen.**

(30) Priorität: **27.05.91 DE 4117252**

(43) Veröffentlichungstag der Anmeldung:
**02.12.92 Patentblatt 92/49**

(45) Bekanntmachung des Hinweises auf die
Patenterteilung:
**06.12.95 Patentblatt 95/49**

(84) Benannte Vertragsstaaten:
**DE ES FR GB IT SE**

(56) Entgegenhaltungen:
**DE-A- 2 001 886    DE-A- 3 905 139**
**DE-B- 1 190 847    GB-A- 2 124 264**
**US-A- 3 717 905    US-A- 4 765 547**

(73) Patentinhaber: **WINKLER & DÜNNEBIER MA-
SCHINENFABRIK UND EISENGIESSEREI KG
Sohler Weg 65
D-56564 Neuwied (DE)**

(72) Erfinder: **Rupp, Heinrich Dipl.-Ing.
Urftstrasse 110
W-4050 Mönchengladbach 2 (DE)**
Erfinder: **Geisen, Armin
Bachstrasse 66
W-5450 Neuwied 22 (DE)**

Rank Xerox (UK) Business Services
(3.10/3.09/3.3.3)

## Beschreibung

Die Erfindung betrifft eine Vorrichtung zum Auflösen von Flockenverklumpungen nach dem Oberbegriff von Anspruch 1.

Bei Maschinen zur Herstellung von weichen, saugfähigen Kissen, wie Damenbinden, Höschenwindeln usw. ist es üblich, auf dem Flockenleger ein Saugkissen aus einem hydrophilen Flockengemisch zu bilden. Die hierzu benötigten weichen, hydrophilen Flocken, vorzugsweise auf Zellulose- und Zellstoffbasis, werden als Flocken-Luftgemisch von einer Zerfaserungseinrichtung kommend durch eine Rohrleitung dem Flockenleger zugeleitet.

Wegen Umwelt- und Lärmschutzbedingungen ist es heute üblich, die Zerfaserungsanlage räumlich getrennt vom Flockenleger anzuordnen und zur Überwindung der größeren Förderdistanz einen Ventilator in die Rohrleitung zu integrieren. In der längeren Rohrleitung kommt es durch Rohrreibung, Strömungsturbulenzen in Verbindung mit statischer Aufladung innerhalb des Flocken-Luftgemisches zu Flockenverdichtungen und Flockenverklumpungen. Dies verhindert einen angestrebten homogenen Flockenaufbau im Saugkissen und beeinträchtigt die Stabilität des Kissens, da Verklumpungen nur in das Flockennetzwerk des Kissens eingelegt, nicht aber eingebunden werden.

Es ist bekannt, zur Lösung dieses Problems, nahe dem Flockenleger einen zusätzlichen angetriebenen Mühlenrotor in die Flockenzuführung zu integrieren. Diese Technik ist z.B. in den US-Patentschriften No. 1,834,309, No. 2,940,133, No. 2,940,134, No. 2,940,135, No. 3,886,629 und No. 4,375,447 beschrieben. Nachteilig bei diesen Lösungen ist, daß sie allesamt teuer sind und einen erheblichen Raumbedarf erfordern. Zudem benötigen diese Rotoren einen Drehantrieb, dessen Drehzahl in Abhängigkeit des Durchsatzes vom Flocken-Luftgemisch angepaßt werden muß. Darüberhinaus erzeugen diese Rotoren unerwünschte Luftverwirbelungen, die einer gleichmäßigen Ausformung des Faserkissens entgegenwirken.

Durch DE-OS 20 01 886 ist außerdem ein Verfahren zum Öffnen von steifen oder starren Fasern bekannt, wie Kohle-, Glas-, Keramikfasern usw. Die Fasern werden mittels eines Gasstromes durch eine Düse geleitet, in der hintereinander einige Prall- und Ablenkplatten als Hindernisse angeordnet sind. Diese verursachen eine gewollte Verwirbelung im Faser-Gasgemisch, wodurch Flockenanhäufungen aufgelöst werden.

Ähnlich arbeitet eine nach US-Pat.-No. 4,765,547 bekannte Vorrichtung, bei der innerhalb einer Kammer und oberhalb einer bodenseitigen, siebartig ausgebildeten Trennwand eine Prallplatte angeordnet ist, auf die von oben ein via einer Düse zugeführter Kohlefaser-Luftstrom mit hoher Geschwindigkeit auftritt. Der senkrecht auf die Prallplatte auftreffende Kohlefaser-Luftstrom wird bei dem Aufprall gewollt verwirbelt. Innerhalb der Verwirbelung lösen sich Faseranhäufungen im Kohlefaser-Luftgemisch und gelangen durch die siebartig ausgebildete Trennwand in eine nachgeordnete Mischkammer.

Sowohl DE-OS 20 01 886 als auch US-Pat.-No. 4,765,547 offenbaren Verfahren bzw. Vorrichtungen, bei denen durch das Einbringen von Hindernissen in einen Faser-Gasstrom, dieser gewollt in einen turbulenten Strömungszustand gebracht wird, wobei sich in den entstehenden Verwirbelungen Faseranhäufungen auflösen. Diese Technik ist allerdings nur beim Öffnen von steifen oder starren Fasern möglich, die zudem in Bezug auf statische Aufladung unproblematisch sind. Beim Transport von weichen Fasern aus Zellstoff-Zellulose usw. in einem Tragluftstrom gilt es jedoch, Luftverwirbelungen zu vermeiden bzw. zu minimieren, da hierdurch die statische Aufladung der Flocken im Flocken-Luftgemisch und somit auch Verklumpungsneigung erhöht wird. Außerdem wird durch die Verwirbelung ein gewünschter homogener Aufbau eines Saugkissens behindert. Zudem rufen die nach der Lehre von DE-OS 20 01 886 und US-Pat.-No. 4,765,547 ausgebildeten und angeordneten Prallplatten, wie Versuche zeigen, Ablagerungen von weichen Flocken auf den Prallflächen hervor, was sehr schnell zu Drosselstellen und schließlich zu Verstopfungen innerhalb der Zuführleitung führt.

Aufgabe der Erfindung ist es, zum Auflösen von Verklumpungen innerhalb eines Flocken-Luftgemisches eine preiswerte kompakte Vorrichtung auszubilden, die aus statischen Elementen aufgebaut ist, welche die Eigendynamik des Flocken-Luftgemisches nutzt und Strömungsturbulenzen weitgehend verhindert, sowie die Nachteile des oben beschriebenen Standes der Technik vermeidet.

Diese Aufgabe wird bei einer gattungsgemäßen Vorrichtung durch die kennzeichnenden Merkmale des Anspruchs 1 gelöst.

Weitere Merkmale der Erfindung gehen aus der Beschreibung sowie den Ansprüchen im Zusammenhang mit der Zeichnung hervor.

Der erzielte Vorteil besteht hauptsächlich darin, daß aus vorwiegend statischen Elementen eine kostengünstige, kompakte Vorrichtung gebildet wird, die zum Auflösen von Flockenverklumpungen die Eigendynamik des Flocken-Luftgemisches nutzt.

Als unerwarteter Vorteil hat sich außerdem herausgestellt, daß im Gegensatz zu allen bekannten Vorrichtungen bei der erfindungsgemäßen Vorrichtung die Arbeitsergebnisse bei gesteigerter Durchsatzmenge des Flocken-Luftgemisches immer besser werden. Als besonders vorteilhaft hat sich erwiesen, daß durch die geneigte Anordnung der Prallelemente in Förderrichtung eine Flocken-Luftstrom Verstopfung verhindert wird.

Die Erfindung wird nachstehend anhand eines Ausführungsbeispiels, das in der Zeichnung dargestellt ist, näher beschrieben.

Es zeigen:

Fig. 1: eine schematische Seitenansicht einer Produktionsanlage zur Herstellung von Saugkissen

Fig. 2: Halbschnitt einer erfindungsgemäßen Pralleinrichtung mit kreisringförmigem Querschnitt

Fig. 3: Draufsicht der Pralleinrichtung mit kreisförmigem Querschnitt

Fig. 4: Schnittdarstellung gemäß Schnitt IV IV in Fig. 2

Fig. 5: Schnittdarstellung gemäß Schnitt V V in Fig. 2

Fig. 6: Seitenansicht einer erfindungsgemäßen Pralleinrichtung in einer Rechteckrohrausführung

Fig. 7: Schnittdarstellung gemäß Schnitt VII VII in Fig. 6

Fig. 8: Schnittdarstellung gemäß Schnitt VIII VIII in Fig. 6

Fig. 9: Schnittdarstellung gemäß Schnitt IX IX in Fig. 6

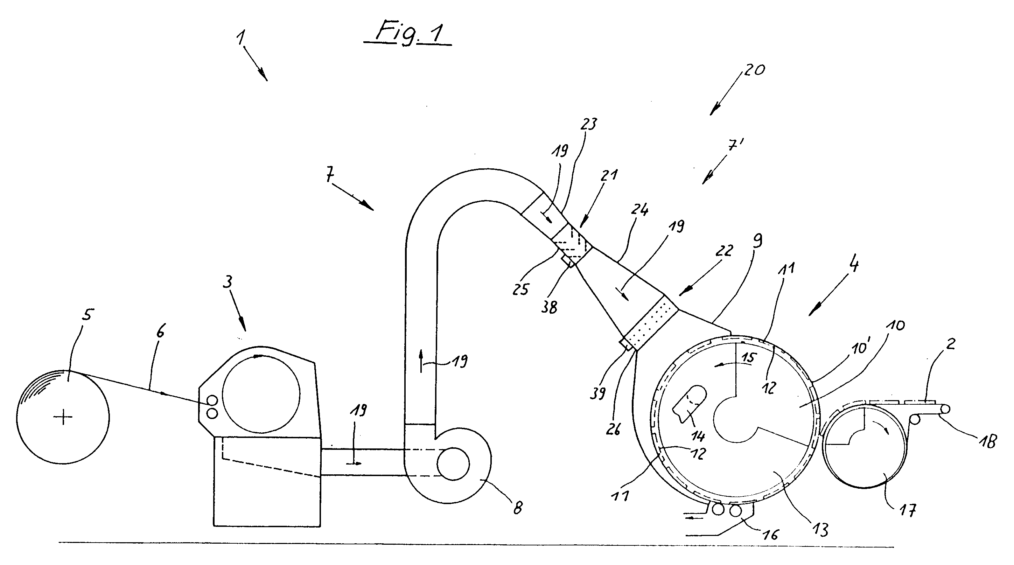

Wie in Fig. 1 gezeigt, sind innerhalb einer Produktionsanlage 1 zur Herstellung von Saugkissen 2 eine Zerfaserungseinrichtung 3 und ein Flockenleger 4 in räumlicher Distanz zueinander angeordnet. Die Zerfaserungseinrichtung 3 wird von einer Vorratsrolle 5 aus mit einer Zellstoffbahn 6 beschichtet. Die Zellstoffbahn 6 wird in der Zerfaserungseinrichtung 3 zu Zellstoff-Flocken aufgelöst, die mit Tragluft versetzt als Flocken-Luftgemisch durch eine Rohrleitung 7 mit Hilfe eines Ventilators 8 einem Flockenkasten 9 des Flockenlegers 4 zugeführt werden. Der Flockenkasten 9 umgreift bogenförmig den Mantel 10' eines rotierenden Formrades 10. Im Mantel 10' sind Formvertiefungen 11 ausgebildet, die siebartigen Böden 12 aufweisen, mittels denen sie mit einem im Innenraum des Formrades 10 angeordneten stationären Saugkasten 13 in luftleitender Verbindung stehen. Mittels des im Saugkasten 13 anstehenden Vakuums, das von einer nicht gezeigten Saugluftquelle via Saugluftleitung 14 bereitgestellt wird, wird das Flocken-Luftgemisch innerhalb des Flockenkastens 9 in die Formvertiefungen 11 angesaugt. Dabei werden die Flocken von ihrer Transportluft befreit und in den Formvertiefungen 11 zu einem Saugkissen 2 verdichtet. In Drehrichtung 15 des Formrades 10 gesehen, ist unmittelbar hinter dem Flockenkasten 9 eine Abbürsteinrichtung 16 installiert, die das über den Mantel 10' aus den Formvertiefungen 11 herausragende Flockenmaterial entfernt und über eine nicht gezeigte Rohrleitung zur Zerfaserungseinrichtung 3 zurückführt. Danach werden die so egalisierten Saugkissen von einer Saugwalze 17 aus den Formvertiefungen 11 entnommen und auf ein Transportband 18 gegeben, welches sie einer nicht gezeigten Produktionsmaschine, z.B. für Damenbinden, Slipeinlagen oder Höschenwindeln, zufördert.

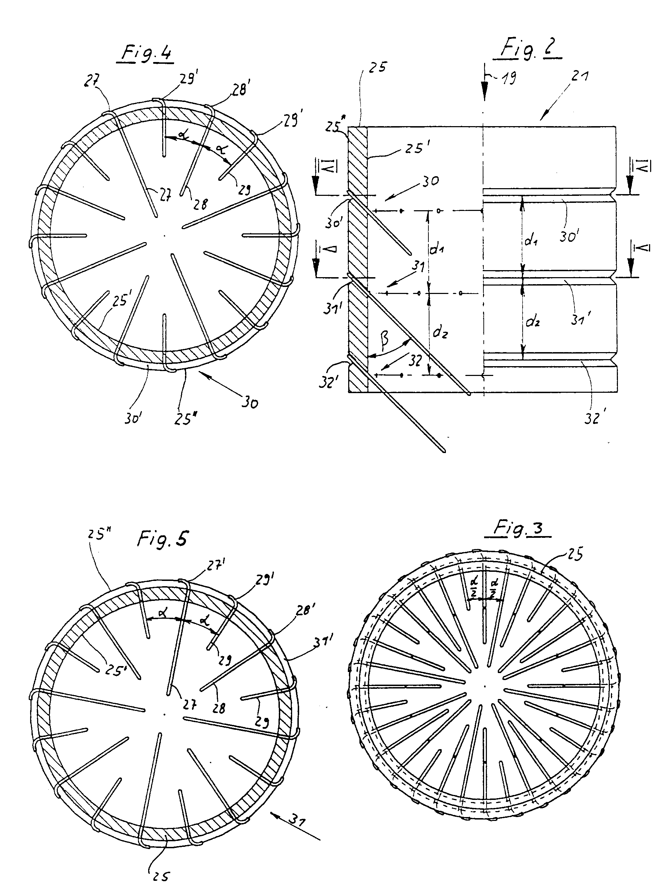

Zur Erreichung einer hohen Flockengüte im Saugkissen 2 ist in die Rohrleitung 7 eine erfinderische Vorrichtung 20 zur Auflösung von Flockenverklumpungen integriert. Die Vorrichtung 20 ist im Endbereich 7' der Rohrleitung 7 vor dem Saugkasten 9 angeordnet und weist eine erste und eine zweite Pralleinrichtung 21,22 auf, die in den Figuren 2 bis 9 näher dargestellt sind.

Zur Erzeugung einer für das Auflösen von Verklumpungen vorteilhaften höheren Strömungsgeschwindigkeit des Flockenluftgemisches, sind den Pralleinrichtungen 21,22 Querschnittsverengungen 23,24 vorgeschaltet.

Die Pralleinrichtung 21 weist als Grundkörper ein rundes Rohr 25 auf, das im Innendurchmesser übergangslos in die vor- und nachgeordneten Querschnittsverengungen 23,24 übergeht und an dessen Innenwand 25' Prallstäbe 27,28,29 kranzförmig in Reihen 30,31,32 angeordnet sind. Die Reihen 30,31,32 weisen untereinander die Abstände $d_1$ und $d_2$ auf. Wie in Fig. 2 gezeigt, sind die Prallstäbe 27,28,29 in Förderrichtung 19 des Flocken-Luftgemisches in einem spitzen Winkel $\beta$ zur Innenwand 25' geneigt und zentrisch zur Mitte des Rohres 25 ausgerichtet. In den Reihen 30,31,32 sind die Prallstäbe 27,28,29 gleichmäßig um einen Winkel $\alpha$ versetzt derart angeordnet, daß auf der Winkelhalbierenden zwischen zwei langen Prallstäben 27 ein halblanger Prallstab 28 angeordnet ist, und daß auf der Winkelhalbierenden zwischen jedem langen und halblangen Prallstab 27,28 jeweils ein kurzer Prallstab 29 eingefügt ist. In Förderrichtung 19 gesehen sind die Reihen 30,31,32 mit ihren Prallstäben 27,28,29 um den Winkel $\frac{\alpha}{2}$ versetzt, so daß die kurzen Prallstäbe 29 der Reihe 30 mit den langen und halblangen Prallstäben 27,28 der Reihe 32 in einer Flucht liegen. Die unterschiedlichen Längen der Prallstäbe 27,28,29 entstehen aus deren zentrischen Anordnung innerhalb des kreisförmigen Rohres 25. Wie in Fig. 3 gezeigt, ergeben sich dadurch in der Projektionsebene aller Prallstäbe 27,28,29 der der Reihen 30,31,32 in etwa gleichbreite Durchflußöffnungen. Diese sind so dimensioniert, daß sie kleiner sind als die größten geduldeten Flockenverklumpungen. Aus Fig. 2 ist ersichtlich, daß die Prallstäbe 27,28,29 in der Wand des Rohres 25 in Bohrungen aufgenommen sind, die von Ringnuten 30',31',32' der Rohraußenseite 25'' aus unter dem Winkel $\beta$ durch die Rohrwand gebohrt sind. Zur Fixierung weisen die Prallstäbe 27,28,29 Abkröpfungen 27',28',29' auf, mit denen sie in den Ringnuten 30',31',32' angelegt sind.

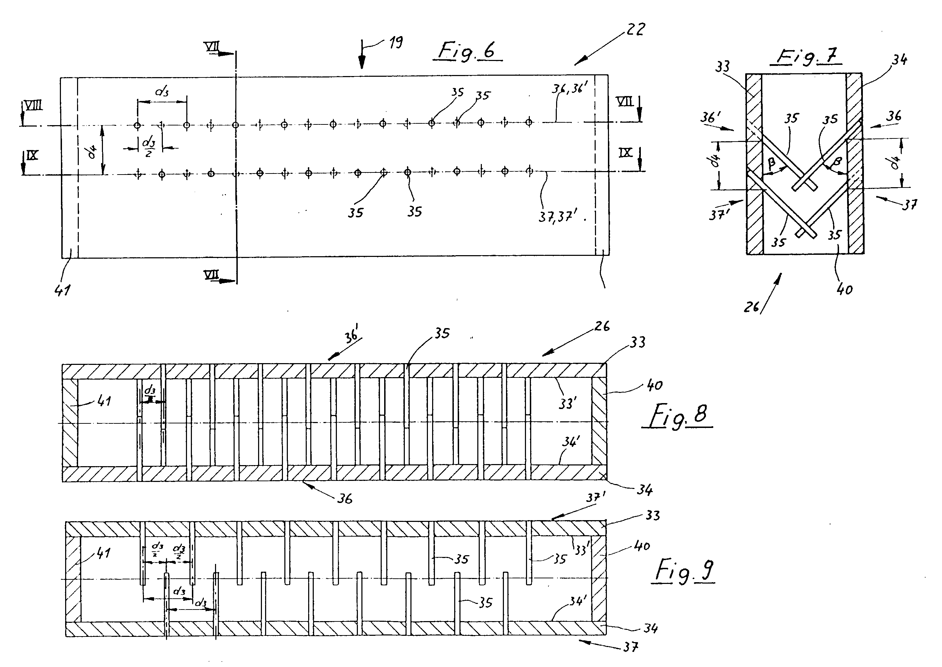

Die Pralleinrichtung 22, detailliert dargestellt in den Fig. 6 bis 9 ist zwischen der Querschnittsverengung 24 und dem Flockenkasten9angeordnet und weist einen Grundkörper in Form eines Rechteckrohres 26 auf. Der dazu notwendige Übergang von einem runden auf einen rechteckigen Querschnitt wird durch entsprechende Ausbildung der Querschnittsverengung 24 erreicht.

An Innenwänden 33',34' von parallelen Längswänden 33,34 des Rechteckrohres 26 sind in gegenüberliegenden Reihen 36,36' bzw. 37,37' gleichlange Prallstäbe 35 angeordnet. Hierbei weisen die Reihen 36,36' einen Abstand $d_4$ zu den Reihen 37,37' auf. Die Prallstäbe 35 sind in Bohrungen befestigt, die in den Längswänden 33,34 angeordnet sind und schließen mit den Innenwänden 33',34', in Förderrichtung 19 gesehen, den Neigungswinkel $\beta$ ein. Innerhalb der Reihen 36,36',37,37' sind die Prallstäbe 35 parallel und im Abstand $d_3$ zueinander ausgerichtet. Die Prallstäbe 35 der Reihen 36,36' bzw. 37,37' sind um $\frac{d3}{2}$ derart versetzt zueinander, daß, wie Fig. 8 zeigt, die Prallstäbe 35 der Reihe 36 die Prallstäbe 35 der Reihe 37' bzw. die Prallstäbe 35 der Reihe 36' die Prallstäbe 35 der Reihe 37 teilweise abdecken. Der Abstand $\frac{d3}{2}$ ist so gewählt, daß er kleiner ist als die größten erlaubten Flockenverklumpungen und daß es trotzdem nicht zu Verstopfungen innerhalb der Pralleinrichtung 22 kommt.

Das Wirkungsprinzip der Vorrichtung 20 zum Auflösen von Flockenverklumpungen besteht darin, daß die Flockenverklumpungen innerhalb des Flocken-Luftgemisches mit dessen Fördergeschwindigkeit auf die Prallstäbe 27,28,29,35 der Pralleinrichtungen 21,22 auftreffen und dabei zerplatzen und aufgelöst werden.

Durch die Anordnung mehrerer Reihen 30,31,32 bzw. 36,36', 37,37' von Prallstäben 27,28,29 bzw. 35 hintereinander wird die Auftreffhäufigkeit von Flockenverklumpungen auf die Prallstäbe 27,28,29,35 erhöht, ohne den freien Strömungsquerschnitt zu verkleinern und ohne den Strömungswiderstand der Pralleinrichtungen 21,22 nennenswert zu erhöhen. Die Abstände $d_1$, $d_2$, $d_4$ sind so groß bemessen, daß die an den stromaufwärtigen Reihen 30,31 bzw. 36,36' abgebremsten Flockenverklumpungen bis zu den nächsten Reihen 31,32 bzw. 37,37' vom Flocken-Luftstrom wieder auf eine Geschwindigkeit beschleunigt werden können, die beim Aufprall auf deren Prallstäbe 27,28,29 bzw. 35 sicher zum Zerplatzen der Flockenverklumpungen führt. Andererseits sind die Abstände $d_1,d_2,d_4$ so knapp bemessen, daß es Flockenverklumpungen nicht erlaubt wird, innerhalb einer laminaren Strömung im Flocken-Luftgemisch ohne Berührung an den Prallstäben vorbeizufließen.Aus dem gleichen Grund ist vor jeder Pralleinrichtung 21 bzw. 22 eine Querschnittsverengung 23 bzw. 24 zur Erhöhung der Strömungsgeschwindigkeit angeordnet. Der Neigungswinkel $\beta$ der Prallstäbe 27,28,29 zur Innenwand 25' bzw. der Prallstäbe 35 zu den Innenwänden 33',34' ist so ausgelegt, daß einerseits im Flocken-Luftstrom Verstopfungen innerhalb der Pralleinrichtungen 21,22 vermieden werden, andererseits den Flockenverklumpungen genügend Widerstand zum Zerplatzen entgegengesetzt wird. Als besonders vorteilhaft haben sich dabei Neigungswinkel $30° < \beta < 55°$ bei Strömungsgeschwindigkeiten zwischen 20m/sek bis 35m/sek herausgestellt.

Zum besseren Verständnis sind vorstehend die Pralleinrichtungen 21,22 in ihrer einfachsten Ausführung beschrieben. Wie in Fig.1 schematisch angedeutet, ist es möglich, die Pralleinrichtungen 21,22 mit einem Schwingungserzeuger 38,39 auszustatten, wodurch die Effizienz der Pralleinrichtung 21,22 und die Flockengüte erhöht wird. Durch die in Vibration versetzten Pralleinrichtungen 21,22 ist es auch möglich, den Neigungswinkel $\beta$ zu vergrößern, ohne daß es hierdurch zu Verstopfungen kommt.

Darüberhinaus ist es sinnvoll, je nach Flockenmaterial und Strömungsgeschwindigkeit die Abstände $d_1,d_2,d_3$ und $d_4$ oder die Winkel $\alpha$ oder $\beta$ zu ändern, gegebenenfalls sogar die Pralleinrichtungen 21,22 auszutauschen. Da die Arbeitsergebnisse bei hohen Strömungsgeschwindigkeiten des Flocken-Luftgemisches immer besser werden, ist der Strömungsquerschnitt der Pralleinrichtung 22 reduzierbar, indem die schmalen Wände 40,41 des Rohres 26 verschiebbar zwischen den Längswänden 33,34 angeordnet sind.

Wie in Fig. 2 und 6 zu sehen, ist der Querschnitt der Prallstäbe 27,28,29,35 aus Kostengründen kreisförmig ausgebildet. In nicht gezeigten aufwendigeren Ausführungen weisen die Prallstäbe einen elliptischen, dreieckig bzw. quadratischen Querschnitt auf.

Es versteht sich von selbst, daß die Erfindung nicht auf die in der Zeichnung dargestellten Ausführungsbeispiele beschränkt ist, da noch mannigfaltige Änderungen und Ergänzungen vorgenommen werden können, ohne daß dadurch vom grundsätzlichen Erfindungsgedanken abgewichen wird.

So ist es zum Beispiel auch möglich, daß die Prallelemente 27,28,29,35 an einer Tragachse oder Tragwand in der Mitte eines Rohrstückes 25 oder 26 befestigt sind und zu den Rohraußenseiten hinzeigen.

**Patentansprüche**

1. Vorrichtung (20) zum Auflösen von Flockenverklumpungen innerhalb eines Flocken-Luftgemisches, das von einer Zerfaserungseinrichtung (3) in Förderrichtung (19) durch eine Rohrleitung (7) einem Flockenleger (4) zur Bildung von Saugkissen (2) zugeführt wird, wobei in einem Endbereich (7') der Rohrleitung (7) mindestens eine Pralleinrichtung (21,22) angeordnet ist, dadurch gekennzeichnet, daß die Pralleinrichtung (21,22) ein Rohrstück (25,26) aufweist, sowie in diesem angeordnete Prallelemente (27,28,29,35), die stabförmig ausgebildet sind und die freie Enden aufweisen, mit denen sie von Innenwänden (25',33',34') der Rohrstücke (25,26) in das Rohrinnere hineinragen, und daß die Prallelemente (27,28,29,35) in Förderrichtung geneigt, in einem spitzen Winkel ( $\beta$ ) zu der Innenwand (25',33',34') angeordnet sind.

2. Vorrichtung nach Anspruch 1, dadurch gekennzeichnet, daß die Prallelemente (27,28,29) einen Abstandswinkel ( $\alpha$ ) aufweisen und in etwa zentrisch zur Rohrmitte hin ausgerichtet sind.

3. Vorrichtung nach Anspruch 1, dadurch gekennzeichnet, daß die Prallelemente (27,28,29,35) einer Pralleinrichtung (21,22) in Reihen (30,31,32,36,36',37,37') an der Innenwand (25', 33',34') angeordnet sind.

4. Vorrichtung nach Anspruch 3, dadurch gekennzeichnet, daß die Reihen (30,31) einen Abstand ($d_1$), die Reihen (31,32) einen Abstand ($d_2$) und die Reihen (36,37) bzw. (36',37') einen Abstand ($d_4$) zueinander aufweisen.

5. Vorrichtung nach Anspruch 1, dadurch gekennzeichnet, daß die Prallelemente (27,28,29) der einzelnen Reihen (30,31,32) in Förderrichtung (19) des Flocken-Luftgemisches gesehen, zueinander um einen Winkel ($\frac{\alpha}{2}$) versetzt angeordnet sind.

6. Vorrichtung nach Anspruch 1, dadurch gekennzeichnet, daß die Länge der Prallelemente (27,28,29) einstellbar ist.

7. Vorrichtung nach Anspruch 1, dadurch gekennzeichnet, daß der Neigungswinkel ( $\beta$ ) einstellbar ist.

8. Vorrichtung nach Anspruch 1, dadurch gekennzeichnet, daß an den Pralleinrichtungen (21,22) ein Schwingungserzeuger (38, 39) angeordnet ist.

9. Vorrichtung nach Anspruch 3, dadurch gekennzeichnet, daß der Abstand ($d_1$,$d_2$,$d_4$) der Reihen 30,31,32,36,36',37,37') veränderbar ist.

10. Vorrichtung nach Anspruch 1, dadurch gekennzeichnet, daß die Pralleinrichtung (21,22) austauschbar ist.

11. Vorrichtung nach Anspruch 1, dadurch gekennzeichnet, daß die Prallelemente (27,28,29,35) unterschiedlich lang sind.

12. Vorrichtung nach Anspruch 1, dadurch gekennzeichnet, daß der Pralleinrichtung (21,22), in Richtung (19) des Flocken-Luftgemisches gesehen, eine Querschnittsreduzierung (23,24) der Rohrleitung (7) zur Erzeugung einer hohen Strömungsgeschwindigkeit vorgeordnet ist.

13. Vorrichtung nach Anspruch 12, dadurch gekennzeichnet, daß die Durchflußöffnung der Pralleinrichtung (21,22) veränderbar ist.

14. Vorrichtung nach Anspruch 8, dadurch gekennzeichnet, daß die Pralleinrichtung (21,22) Drehschwingungen ausführt.

15. Vorrichtung nach Anspruch 8, dadurch gekennzeichnet, daß die Prallelemente (27,28,29,35) in einer wählbaren Frequenz vibrieren.

16. Vorrichtung nach Anspruch 3, dadurch gekennzeichnet, daß die Prallelemente (35) in der Reihe (36,36',37,37') einen Abstand ($d_3$) aufweisen.

17. Vorrichtung nach Anspruch 16, dadurch gekennzeichnet, daß in Förderrichtung (19) gesehen die Reihen (36) zu 36') und (37) zu (37') um einen Abstand ($\frac{d3}{2}$) versetzt sind.

**Claims**

1. Apparatus (20) for breaking up flock lumps within a flock-air mixture, which is supplied from a disintegration device (3) in a conveying direction (19) through a conduit (7) to a flock layering apparatus (4) for forming absorbent pads (2), at least one impingement device (21,22) being arranged in an end region (7') of the conduit (7), characterised in that the impingement device (21,22) has a pipe portion (25,26) and impingement elements (27,28,29,35) arranged in it which are of rod-shaped construction and which have free ends which project from interior walls (25',33',34') of the pipe portions (25,26) into the interior of the pipe and that the impingement elements (27,28,29,35) are arranged inclined in the conveying direction at an acute angle ($\beta$) to the interior wall (25',33',34').

2. Apparatus as claimed in claim 1, characterised in that the impingement elements (27,28,29) have a spacing angle ($\alpha$) and are aligned approximately centrally towards the centre of the pipe.

3. Apparatus as claimed in claim 1, characterised in that the impingement elements (27,28,29,35) of an impingement device (21,22) are arranged in rows (30,31,32,36,36',37,37') on the interior wall (25',33',34').

4. Apparatus as claimed in claim 3, characterised in that the rows (30,31) have a spacing ($d_1$) from one another, the rows (31,32) have a spacing ($d_2$) from one another and the rows (36,37) and (36',37') have a spacing ($d_4$) from one another.

5. Apparatus as claimed in claim 1, characterised in that the impingement elements (27,28,29) of the individual rows (30,31,32) are arranged offset from one another by an angle ($\alpha/2$), seen in the conveying direction (19) of the flock-air mixture.

6. Apparatus as claimed in claim 1, characterised in that the length of the impingement elements (27,28,29) is adjustable.

7. Apparatus as claimed in claim 1, characterised in that the angle of inclination ($\beta$) is adjustable.

8. Apparatus as claimed in claim 1, characterised in that a vibration generator (38,39) is arranged on the impingement devices (21,22).

9. Apparatus as claimed in claim 3, characterised in that the spacing ($d_1,d_2,d_4$) of the rows (30,31,32,36,36', 37,37') is alterable.

10. Apparatus as claimed in claim 1, characterised in that the impingement device (21,22) is interchangeable.

11. Apparatus as claimed in claim 1, characterised in that the impingement elements (27,28,29) are of different length.

12. Apparatus as claimed in claim 1, characterised in that a cross-sectional reduction (23,24) of the conduit (7) for producing a high flow velocity is arranged upstream of the impingement device (21,22), seen in the direction (19) of the flock-air mixture.

13. Apparatus as claimed in claim 12, characterised in that the flow opening of the impingement device (21,22) is alterable.

14. Apparatus as claimed in claim 8, characterised in that the impingement device (21,22) performs rotational oscillations.

15. Apparatus as claimed in claim 8, characterised in that the impingement elements (27,28,29,35) vibrate at a selectable frequency.

16. Apparatus as claimed in claim 3, characterised in that the impingement elements (35) in the row (36,36',37,37') have a spacing ($d_3$).

17. Apparatus as claimed in claim 16, characterised in that the rows (36') to (36') and (37) to (37') are offset by a distance ($d_3/2$), seen in the conveying direction (19).

**Revendications**

1. Dispositif (20) destiné à désagréger des masses de flocons dans un mélange de flocons en suspension dans l'air, qui est transporté dans un conduit (7), dans le sens d'écoulement (19), d'un appareil effilocheur (3) vers un appareil de pose (4) pour former des tampons absorbants (2), au moins un dispositif (21, 22) à chicanes étant implanté à une extrémité (7') de ce conduit, dispositif caractérisé par le fait que le dispositif (21, 22) à chicanes comporte un tronçon de tube (25, 26) et des éléments de chicane (27, 28, 29, 35) qui y sont logés, ont la forme de barreaux, présentent une extrémité libre, partent des parois intérieures (25', 33', 34') du tube (25, 26) et sont orientés vers l'intérieur de ce tube (25, 26), lesdits éléments de chicane (27, 28, 29, 35) étant inclinés, dans le sens de l'écoulement du mélange, d'un angle aigu ($\beta$) sur la paroi intérieure respective (25', 33', 34').

2. Dispositif selon la revendication 1, caractérisé par le fait que les éléments de chicane (27, 28, 29) sont espacés d'un angle ($\alpha$) et sont orientés sensiblement vers l'axe du tube (25).

3. Dispositif selon la revendication 1, caractérisé par le fait que les éléments de chicane (27, 28, 29, 35) d'un dispositif (21, 22) à chicanes sont agencés en des rangées (30, 31, 32 ; 36, 36' ; 37, 37') sur la paroi intérieure (25', 33', 34').

4. Dispositif selon la revendication 3, caractérisé par le fait que les rangées (30, 31) sont espacées d'une distance ($d_1$), les rangées (31, 32) d'une distance ($d_2$), et les rangées respectives (36, 37) ou (36', 37') d'une distance ($d_4$).

5. Dispositif selon la revendication 1, caractérisé par le fait que les éléments de chicane (27, 28, 29) de chaque rangée (30, 31, 32), vus dans le sens (19) du passage du mélange de flocons et d'air, sont décalés entre eux d'un angle ($\frac{\alpha}{2}$).

6. Dispositif selon la revendication 1, caractérisé par le fait que la longueur des éléments de chicane (27, 28, 29) est réglable.

7. Dispositif selon la revendication 1, caractérisé par le fait que l'angle d'inclinaison ($\beta$) est réglable.

8. Dispositif selon la revendication 1, caractérisé par le fait qu'un générateur de vibrations (38, 39) est monté sur les dispositifs (21, 22) à chicanes.

9. Dispositif selon la revendication 3, caractérisé par le fait que la distance ($d_1$, $d_2$, $d_4$), comprise entre les rangées (30, 31, 32, 36, 36' ; 37, 37'), peut être modifiée.

10. Dispositif selon la revendication 1, caractérisé par le fait que les dispositifs (21, 22) à chicanes sont remplaçables.

11. Dispositif selon la revendication 1, caractérisé par le fait que les éléments de chicane (27, 28, 29, 35) ont des longueurs différentes.

12. Dispositif selon la revendication 1, caractérisé par le fait que le dispositif (21, 22) à chicanes présente,en amont, un rétrécissement (23, 24) du conduit (7), dans le sens (19) du passage du mélange de flocons et d'air, en vue d'augmenter la vitesse d'écoulement.

13. Dispositif selon la revendication 12, caractérisé par le fait que l'orifice de passage du dispositif (21, 22) à chicanes peut être modifié.

14. Dispositif selon la revendication 8, caractérisé par le fait que le dispositif (21, 22) à chicanes exécute des vibrations tournantes.

15. Dispositif selon la revendication 8, caractérisé par le fait que les éléments de chicane (27, 28, 29, 35) vibrent à une fréquence sélectionnable.

16. Dispositif selon la revendication 3, caractérisé par le fait que les éléments de chicane (35) sont espacés d'une distance ($d_3$) dans la rangée (36, 36', 37, 37').

17. Dispositif selon la revendication 16, caractérisé par le fait que les rangées (36 et 36') et (37 et 37') sont respectivement décalées d'une distance ($\frac{d_3}{2}$ ) observées dans le sens (19) de passage du mélange.

Fig. 1

EP 0 515 939 B1

Fig. 4

Fig. 2

Fig. 5

Fig. 3

Fig. 6

Fig. 7

Fig. 8

Fig. 9

EP 0 515 939 B1